(19) Europäisches Patentamt
European Patent Office
Office européen des brevets

(11) Numéro de publication: **0 010 030**
**B1**

(12) # FASCICULE DE BREVET EUROPEEN

(45) Date de publication du fascicule de brevet: **05.05.82**

(51) Int. Cl.³: **C 07 C 103/50**, C 07 D 295/14, A 61 K 31/16, A 61 K 31/395

(21) Numéro de dépôt: **79400668.4**

(22) Date de dépôt: **20.09.79**

(54) **Nouveaux dérivés d'ortho chloro benzoyl-2 chloro-4 glycylanilide, leur préparation et leur application en tant que médicaments.**

(30) Priorité: **25.09.78 FR 7827401**

(43) Date de publication de la demande: **16.04.80 Bulletin 80/8**

(45) Mention de la délivrance du brevet: **05.05.82 Bulletin 82/18**

(84) Etats contractants désignés: **AT BE CH DE FR GB IT LU NL SE**

(56) Documents cités:

**EP-A-0 000 299**
**FR-M-1 397**
**GB-A-985 683**
**US-A-3 950 383**

**CHEMICAL ABSTRACTS, vol. 88, No. 15, 10 avril 1978, page 530, ref. 104908k Columbus, Ohio, US**

(73) Titulaire: **PIERRE FABRE S.A., 125, rue de la Faisanderie, F-75116 Paris (FR)**

(72) Inventeur: **Mouzin, Gilbert, 21, rue Sainte Foy, F-81000 Castres (FR)**
Inventeur: **Cousse, Henri, Chemin de Lastinos, F-81000 Castres (FR)**
Inventeur: **Stenger, Antoine, 11, rue des Cigales, F-81000 Castres (FR)**

(74) Mandataire: **Corre, Jacques et al, Cabinet Regimbeau 26, Avenue Kléber, F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG.

# Nouveaux dérivés d'ortho chloro benzoyl-2 chloro-4 glycylanilide, leur préparation et leur application en tant que médicaments

La présente invention, réalisée au Centre de Recherches Pierre FABRE, concerne de nouveaux dérivés d'ortho chloro benzoyl-2 chloro-4 glycylanilides substitués, leur procédé de préparation et leur application en tant que médicaments.

Dans la technique antérieure la plus proche, par exemple illustrée par le Brevet Spécial de Médicament français n° 1397 M, on a déjà proposé, à titre de médicaments doués de propriétés anti-convulsives et tranquillisantes, des dérivés de 2-amino-acétamido-benzophénones.

Il s'est avéré cependant que de tels composés à structure ouverte présentent une très grande instabilité et ont spontanément tendance à se cycliser pour donner naissance à des composés à structure benzodiazépinique fermée.

Les travaux de Von H. Oelschläger & Coll. (Arzneimittel Forsch. 1973, pages 802 et suivantes) ont ensuite révélé que les benzophénones à structure ouverte étaient inactives et que la présence d'un cycle diazépinique était la condition sine qua non pour obtenir une activité.

La présente invention se rapporte en revanche à des dérivés se distinguant, de ceux de la technique antérieure précitée, par une substitution sur le premier atome d'azote en série glycylanilidique, répondant à la formule:

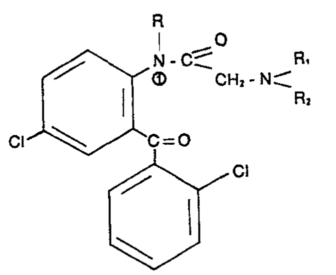

Or, il a été démontré que cette substitution en série glycylanilidique influençait la distance N-N, laquelle apparaît comme étant un paramètre déterminant pour l'activité de ce type de composés.

Il convient de noter que cette constatation est tout à fait surprenante, compte tenu du fait que dans le cas de composés benzodiazépiniques il n'existe qu'une faible différence d'activité entre le diazépam et le diméthyldiazépam, alors qu'en série glycylanilidique la différence d'activité s'est révélée très importante.

Les nouveaux dérivés de la présente invention répondent à la formule générale I:

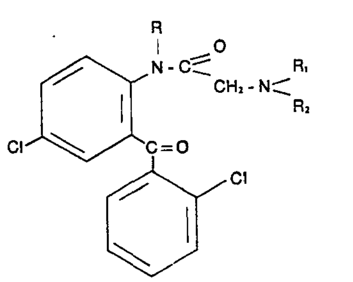

(I)

dans laquelle:

- R représente un groupe alcoyle inférieur et plus particulièrement le radical méthyle;
- $R_1$ représente un hydrogène, un alcoyle, ou un hydroxyalcoyle;
- $R_2$ représente un hydroxyalcoyle, un alcényle, un alcynyle éventuellement substitués une ou plusieurs fois par un radical alcoyle, ou un cycloalcoyle de 3 à 6 atomes de carbone éventuellement substitué par un radical alcoyle;
- $R_1$ et $R_2$ peuvent également former avec l'atome d'azote auquel ils sont fixés un hétérocycle azoté contenant éventuellement un second hétéroatome choisi parmi l'oxygène et l'azote;

ainsi que leurs sels obtenus avec des acides minéraux ou organiques thérapeutiquement acceptables.

On indiquera ci-après une explication illustrative de quelques significations données à propos des radicaux R, $R_1$ et $R_2$. Les groupes alcoyle désignent des groupes alcoyle à chaîne linéaire ou ramifiée, tels que méthyle, éthyle, propyle, isopropyle, butyle, insobutyle, sec.-butyle, tert.-butyle, pentyle, isopentyle, néopentyle, tert.-pentyle, hexyle, isohexyle, heptyle, etc. Les groupes alcényle peuvent être choisis parmi les groupes allyle, butényle, pentadiényle etc. Les groupes alcynyle peuvent être choisis parmi les groupes éthynyle, propargyle, etc. Enfin, les hétérocycles saturés ou non sont choisis par exemple parmi les groupes pyrrolyle, imidazolyle, pyrazolyle, isoxazolyle, pyridyle, pyrazinyle, pyrinidinyle, pyridazinyle, pyrrolidinyle, imidazolidinyle, pyrazolidinyle, pipéridyle, pipérazinyle, morpholinyle, etc.

La présente invention s'applique également aux sels des composés de formule (I) obtenus avec des acides thérapeutiquement acceptables.

A titre d'exemples non limitatifs de sels d'addition thérapeutiquement ou physiologiquement acceptables, on peut citer les sels d'acides minéraux, tels que les acides chlorhydrique, phosphorique et sulfurique, et les sels d'acides organiques, tels que les acides maléique, succinique, fumarique, citrique, etc.

La présente invention concerne également un procédé de préparation des composés de formule I, caractérisé en ce que l'on condense un composé de formule générale II:

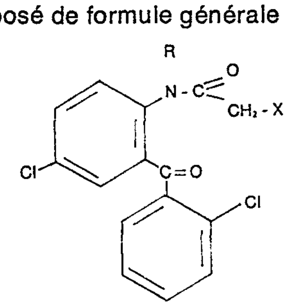

(II)

dans laquelle:

R a la signification donnée à propos de la formule générale I, et

X représente un atome d'halogène avec une amine de formule générale III:

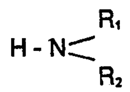

(III)

dans laquelle:

$R_1$ et $R_2$ ont la signification donnée à propos de la formule générale I.

Les intermédiaires de synthèse de formule générale II peuvent être préparés à partir d'amino benzophénones obtenues selon L. H. STERNBACH et R. Ian FRYER - J. Org. Chem. 27, 3781, 1962, ou encore conformément au procédé du brevet français n° 7 826 918 déposé au nom de la demanderesse, correspondant au schéma réactionnel suivant:

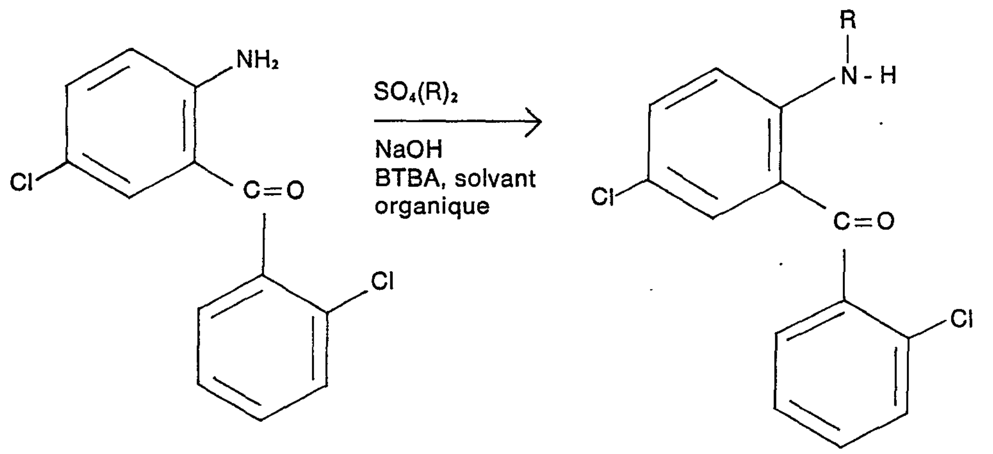


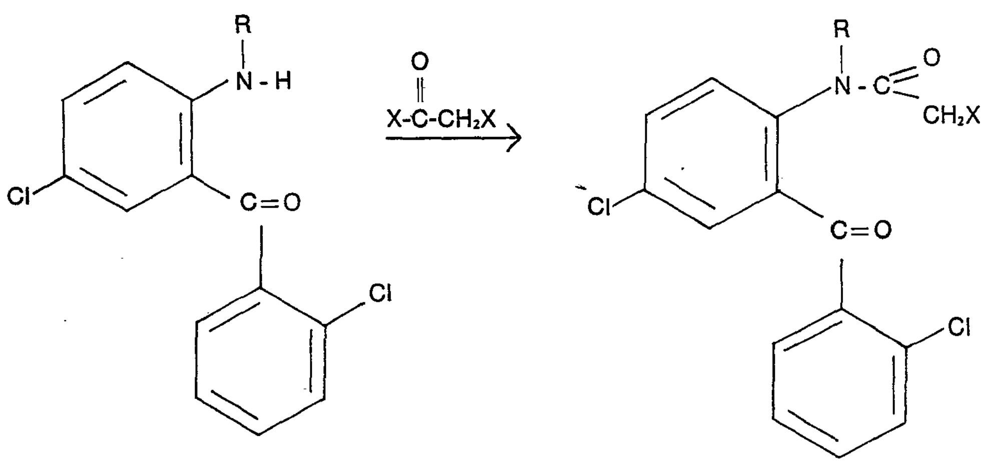

X représente un atome d'halogène et R a la signification donnée précédemment.

La présente invention concerne enfin l'application des composés de formule générale I en tant que médicaments doués d'une activité sur le système nerveux central et en particulier en tant qu'agents anxiolytiques, sédatifs, anti-convulsifs, hypnotiques ou comme agent de relaxation musculaire.

La présente invention sera décrite ci-après plus en détail à propos des quelques exemples non limitatifs suivants:

Exemple 1

N-N' diméthyl N(hydroxy-2 éthyl) ortho chloro benzoyl-2' chloro-4' glycylanilide

a) Préparation du N-méthyl benzoyl-2' dichloro-2"-4' bromo-2 acétanilide

A une solution de 56 g de méthyl amino-2 dichloro-2'-5 benzophénone dans 400 $cm^3$ d'acétate d'éthyle on ajoute un volume égal de glace, puis 23 $cm^3$ de bromure de bromacétyle. Après une nuit d'agitation à température ambiante, on ajoute 300 $cm^3$ d'éther éthylique, on décante, on lave à la soude 2 N et on lave à l'eau jusqu'à neutralité.

On sèche ensuite sur sulfate de sodium, on filtre et l'on évapore jusqu'à siccité. Le résidu est traité à l'éther de pétrole puis recristallisé dans l'acétate d'éthyle. On récupère ainsi 65 g de cristaux, rendement 81%.

Point de fusion: 86°C.

Chromatographie sur plaque:

support: gel de silice 60 F 254 Merck

solvant: acétate d'éthyle - éther pe pétrole 30/70

révélation: UV et iode

Rf: 0,33

b) Préparation de N-N' diméthyl N(hydroxy-2 éthyl) ortho chloro benzoyl-2' chloro-4' glycylanilide

A une solution de 60,15 g (0,15 mole) de N-méthyl ortho chloro benzoyl-2' chloro-4' bromo-2 acétanilide dans 500 cm³ d'acétone, on ajoute 25 cm³ (0,31 mole) de méthyl amino-2 éthanol et l'on chauffe 2 heures à reflux.

On évapore la solution jusqu'à siccité, on reprend le résidu à l'acide chlorhydrique N et l'on lave à l'éther. La phase aqueuse acide est traitée au bicarbonate de sodium, puis on extrait à l'éther et l'on lave à l'eau jusqu'à neutralité.

On sèche sur sulfate de sodium, on filtre et l'on évapore la phase organique. Le résidu obtenu est recristallisé dans l'hexane-acétate de éthyle. On récupère ainsi, avec un rendement de 80%, le produit de formule:

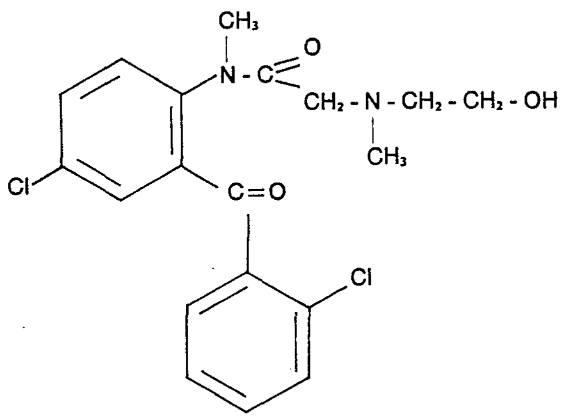

Formule brute: $C_{19}H_{20}Cl_2N_2O_3$
Masse moléculaire: 395,27
Cristaux: blanc cassé
Point de fusion: 79°C
Chromatographie sur plaque:
support: gel de silice 60 F 254 Merck
solvant: butanol-acide acétique-eau 6/2/2
révélation: UV et iode
Rf: 0,41

Exemple 2

N-N bis (hydroxy-2 éthyl)N' méthyl ortho chloro benzoyl-2' chloro-4' glycylanilide, chlorhydrate

A une solution de 40,1 g (0,1 mole) de N-méthyl ortho chloro benzoyl-2' chloro-4' bromo-2 acétanilide dans 300 cm³ d'acétone, on ajoute 20 cm³ (0,2 mole) de diéthanolamine et on agite 24 heures à température ambiante.

On évapore jusqu'à siccité le solvant réactionnel, on traite le résidu par une solution bicarbonatée et l'on extrait à l'acétate d'éthyle. On lave la phase organique 3 fois à l'eau et l'on sèche sur sulfate de sodium.

Après filtration et évaporation, on récupère 43 g d'huile que l'on traite par une solution éthanolique saturée d'acide chlorhydrique; on précipite à l'éther éthylique et l'on glace. On récupère après filtration et séchage 32 g de cristaux. Rendement 70% en produit de formule:

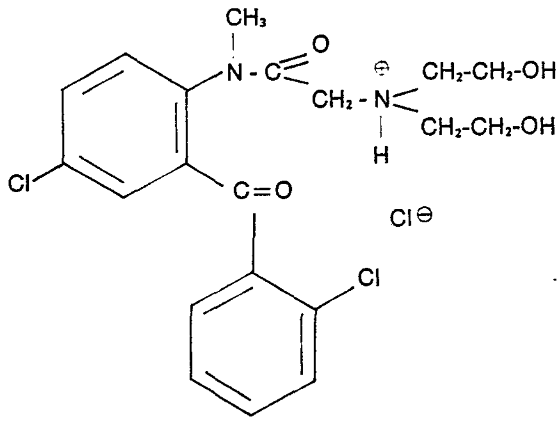

Formule brute: $C_{20}H_{23}Cl_3N_2O_4$
Masse moléculaire: 461,77
Cristaux blancs
Point de fusion: 187-188°C
Chromatographie sur plaque:
support: gel de silice 60 F 254 Merck
solvant: butanol-acide acétique-eau 6/2/2
révélation: UV et iode
Rf: 0,35

Exemple 3

N(diméthyl-1-1 propargyl) N' méthyl ortho chloro benzoyl-2' chloro-4' glycylanilide, chlorhydrate

A une solution de 7,37 g (0,09 mole) de diméthyl-1-1 propargylamine dans 30 cm³ d'acétone, on ajoute 4,01 g de N-méthyl ortho chloro benzoyl-2' chloro-4' bromo-2 acétanilide. Après 5 heures à température ambiante, on évapore l'acétone, on traite le résidue par une solution bicarbonatée et l'on extrait à l'acétate d'éthyle.

Après les traitements habituels l'huille résiduelle est traitée par une solution éthanolique saturée d'acide chlorhydrique.

On récupère avec un rendement de 75%, 3,29 g de cristaux de formule:

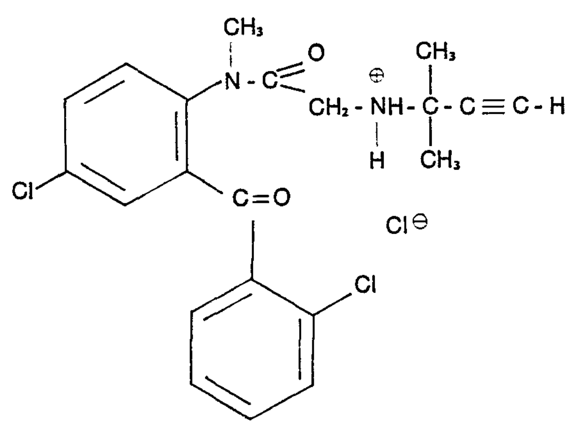

Formule brute: $C_{21}H_{21}Cl_3N_2O_2$
Masse moléculaire: 439,77
Cristaux blancs
Point de fusion: 176°C
Chromatographie sur plaque:
support: gel de silice 60 F 254 Merck
solvant: butanol-acide acétique-eau 6/2/2
révélation: UV et iode
Rf: 0,62

Exemple 4

N-cyclopropyl N'-méthyl ortho chloro benzoyl-2' chloro-4' glycylanilide, chlorhydrate

A une solution de 3,15 g (0,0078 mole) de N-méthyl benzoyl-2' dichloro-4'-2" bromo-2 acétanilide dans 25 cm³ de chlorure de méthylène, on ajoute 1,6 cm³ (0,0231 mole) de cyclopropylamine. On agite 4 heures à température ambiante, on évapore le solvant et l'on traite le résidu par une solution bicarbonatée.

On extrait à l'acétate d'éthyle, on décante, on lave à l'eau et l'on sèche sur sulfate.

Après filtration et évaporation, l'huile résiduelle obtenue est traitée par une solution éthanolique saturée d'acide chlorhydrique.

On récupère ainsi avec un rendement de 85% le produit de formule:

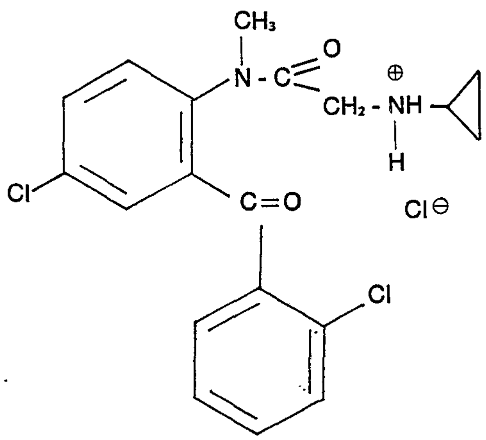

Formule brute: $C_{19}H_{19}Cl_3N_2O_2$
Masse moléculaire: 413,73
Cristaux blancs
Point de fusion: 201°C
Chromatographie sur plaque:
support: gel de silice 60 F 254 Merck
solvant: butanol-acide acétique-eau 6/2/2
révélation: UV et iode
Rf: 0,53
Solubilité: soluble dans l'eau à 0,3%.

Exemple 5

N-cyclopentyl N'-méthyl ortho chloro benzoyl-2' chloro-4' glycylanilide, chlorhydrate

D'une manière analogue à celle de l'exemple 4, mais en utilisant la cyclopentylamine, on obtient le produit de formule:

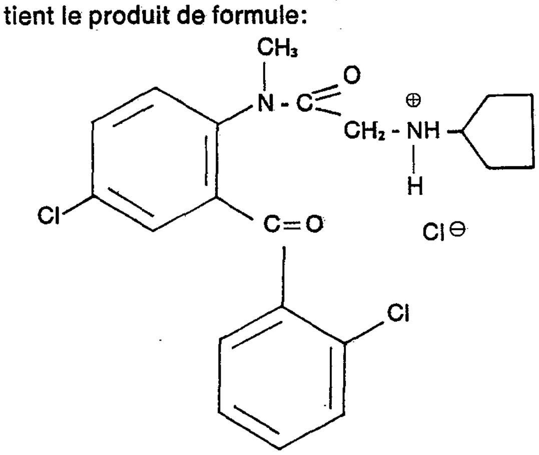

Formule brute: $C_{21}H_{23}Cl_3N_2O_2$
Masse moléculaire: 441,79
Cristaux blancs
Point de fusion: 210°C
Chromatographie sur plaque:
support: gel de silice 60 F 254 Merck
solvant: méthanol-chloroforme 50/50
révélation: UV et iode
Rf: 0,78
Solubilité: soluble dans l'eau à 0,5%.

Exemple 6

N-cyclohexyl N' méthyl ortho chloro benzoyl-2' chloro-4' glycylanilide, maléate acide

D'une manière analogue à celle décrite dans l'exemple 4, mais en utilisant la cyclohexylamine comme amine et l'acide maléique comme agent salifiant, on obtient le produit de formule:

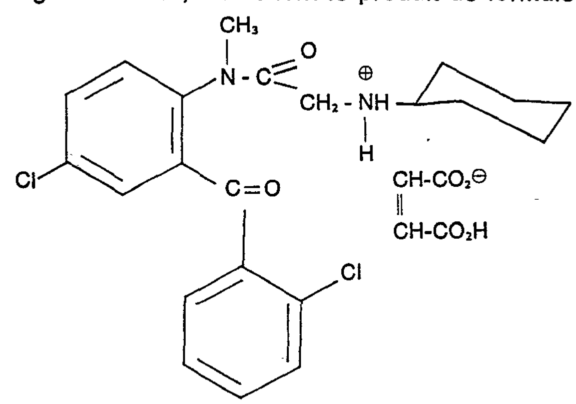

Formule brute: $C_{26}H_{28}Cl_2N_2O_6$
Masse moléculaire: 535,43
Cristaux blancs
Point de fusion: 150°C
Chromatographie sur plaque:
support: gel de silice 60 F 254 Merck
solvant: butanol-acide acétique-eau 6/2/2
révélation: UV et iode
Rf: 0,63
Solubilité: Soluble à 1% dans le propylène glycol. Insoluble dans l'eau. Soluble à 10% dans le DMA.

Exemple 7

N-cyclohexyl N-N' diméthyl ortho chloro benzoyl-2' chloro-4' glycylanilide, maléate acide

D'une manière analogue à celle décrite dans l'exemple 4, mais en utilisant la N-méthyl cyclohexylamine comme amine et l'acide maléique comme agent salifiant, on obtient le produit de formule:

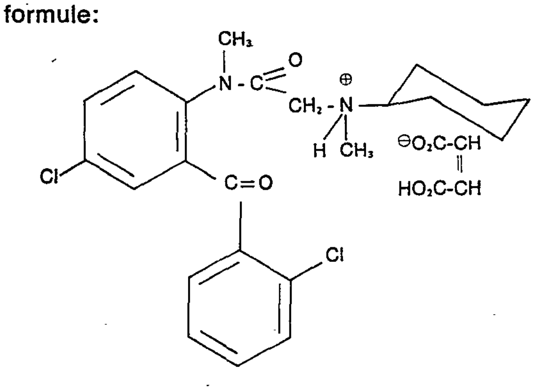

Formule brute: $C_{27}H_{30}Cl_2N_2O_6$
Masse moléculaire: 549,45
Cristaux blancs
Point de fusion: 192°C
Chromatographie sur plaque:
support: gel de silice 60 F 254 Merck
solvant: butanol-acide acétique-eau 6/2/2
révélation: UV et iode
Rf: 0,41
Solubilité: Insoluble dans l'eau. Soluble à 5% dans le DMSO.

Exemple 8
N-méthyl ortho chloro benzoyl-2' chloro-4' morpholino-2 acétanilide, maléate acide

D'une manière analogue à celle décrite dans l'exemple 4, mais en utilisant la morpholine comme amine et l'acide maléique comme agent salifiant on obtient le produit de formule:

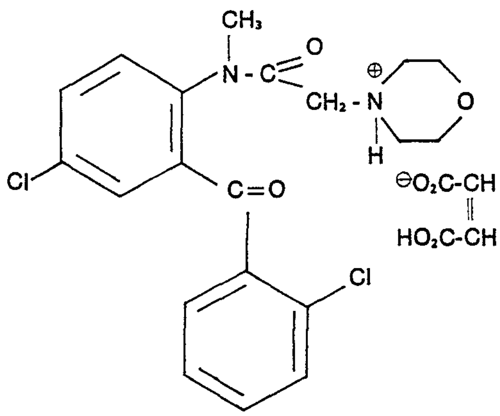

Formule brute: $C_{24}H_{24}Cl_2N_2O_7$
Masse moléculaire: 523,37
Cristaux blancs
Point de fusion: 125°C
Chromatographie sur plaque:
support: gel de silice 60 F 254 Merck
solvant: butanol-acide acétique-eau 6/2/2
révélation: UV et iode
Rf: 0,50
Solubilité: soluble dans l'eau à 1%.

Exemple 9
N-méthyl N'(méthyl-2 allyl) ortho chloro benzoyl-2' chloro-4' glycylanilide

D'une manière analogue à celle décrite dans l'exemple 4, mais en utilisant la méthyl allylamine, on obtient le produit de formule:

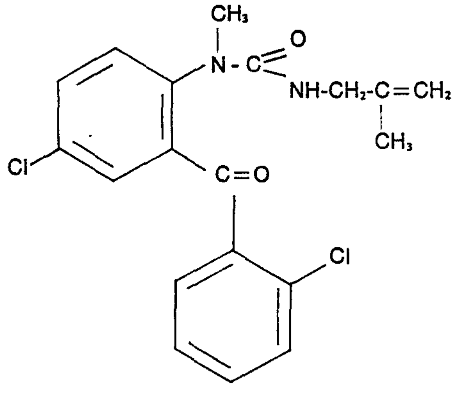

Formule brute: $C_{20}H_{20}Cl_2N_2O_2$
Masse moléculaire: 391,3
Cristaux blancs
Chromatographie sur plaque:
support: gel de silice 60 F 254 Merck
solvant: butanol-acide acétique-eau 6/2/2
révélation: UV et iode
Rf: 0,67.

Les composés de la présente invention, doués d'une remarquable activité sur le système nerveux central, sont donc susceptibles d'être administrés à l'homme ou à l'animal par voie orale ou par injection, sous la forme d'une base libre ou bien de l'un de ses sels thérapeutiquement acceptables.

A titre de simple illustration on indiquera ci-après quelques rétultats des divers essais toxicologiques et pharmacologiques effectués sur les composés de l'invention.

a) Etude de toxicité

Les composés de la présente invention ont été soumis à des contrôles de toxicité. La toxicité de certains composés déterminée par la dose létale 50 est rapportée dans le tableau suivant. Elle a été recherchée sur des lots de 10 souris par voie orale, et calculée selon la méthode de MILLER et TAINTER (Proc. Soc. expér. Biol. Méd., 1944, 57, 261).

b) Activité dans l'essai sur la tige tournante (Rota-Rod)

On réalise cet essai sur des souris mâles de souche Swiss.

On place la souris sur une tige de bois d'un diamètre de 3 cm, tournant à raison de 5 tours par minute. On choisit les souris qui peuvent rester sur la tige pendant au moins 3 minutes au cours d'essais successifs et on les rassemble par groupes de 10 pour l'essai de chaque dose.

Si la souris tombe de la tige en moins de deux minutes, on considère le composé essayé comme efficace.

Les résultats sont exprimés en $DE_{50}$ selon: N. W. DUNHAM et T. S. MIVA (J. Amer. pharm. Asso., 1957, 46, 208).

c) Activité antagoniste au pentétrazol

Ce test est réalisé sur un groupe de 10 souris mâles de souche Swiss. Dans un délai de 15 minutes après l'injection sous-cutanée de 125 mg/kg de pentétrazol, les souris ont des convulsions toniques dont l'issue est fatale. Pour l'essai, on administre par voie orale les composés 60 minutes avant l'injection de pentétrazol. Les animaux sont observés pendant 2 heures après administration du pentétrazol.

Les résultats sont exprimés par la dose efficace $DE_{50}$ selon GOODMANN et coll. (J. Pharmacol. 108, 1953).

TABLEAU DE RESULTATS

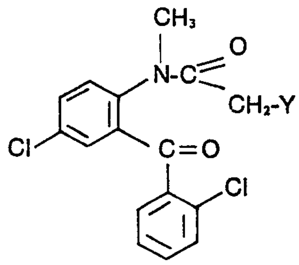

| Y | Toxicité v.o. $DE_{50}$ mg/kg | Rota Rod v.o. $DE_{50}$ mg/kg | Pentétrazol v.o. $DE_{50}$ mg/kg |
|---|---|---|---|
| -N-$CH_2$-$CH_2OH$<br>$CH_3$ | 750 | 17 | 0,9 |
| $CH_2$-$CH_2OH$<br>-N<br>$CH_2$-$CH_2OH$ | ≃1000 | 15 | 0,5 |
| $CH_3$<br>-N-C-C≡C-H<br>H $CH_3$ | 420 | 19 | 1 |
| 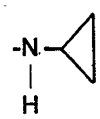 | >1000 | 7 | 0,3 |
| 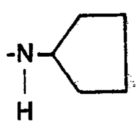 | 750 | 16 | 1,9 |
| 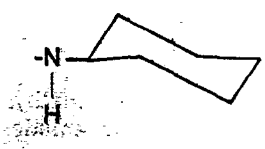 | >1000 | 20 | 0,7 |
| 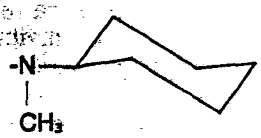 | >1000 | 21 | 0,8 |
| 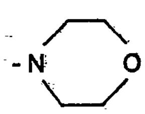 | >1000 | 20 | 0,8 |
| -N-$CH_2$-C=$CH_2$<br>H $CH_3$ | >1000 | 10 | 0,4 |

Compte tenu de leurs propriétés pharmacologiques et de leur faible toxicité, ces composés chimiques peuvent être utilisés en thérapeutique dans le traitement de l'anxiété et des névroses.

Ces composés et leurs sels d'addition d'acides thérapeutiquement compatibles peuvent être utilisés comme médicaments, par exemple sous forme de préparations pharmaceutiques adaptées à l'administration entérale ou parentérale, avec par exemple, l'eau, le lactose, la gélatine, les amidons, le stéarate de magnésium, le talc, les huiles végétales, les gommes, les polyalcoylèneglycols, la vaseline, etc. Ces préparations peuvent se présenter sous forme solide, par exemple de comprimés, dragées, capsules, etc. ou sous forme liquide, par exemple de solutions, suspensions ou émulsions.

Les préparations pharmaceutiques sous une forme appropriée à l'injection sont préférées. Ces préparations peuvent être soumises à des opérations pharmaceutiques classiques telles que stérilisation et/ou peuvent contenir des adjuvants par exemple des agents conservateurs, stabilisants, de mouillage ou d'émulsification, des composés-tampons, etc.

Les dosages, auxquels les composés actifs et leurs sels d'addition d'acide thérapeutiquement compatibles peuvent être administrés, peuvent varier dans des proportions importantes selon l'état du patient. Un dosage quotidien de environ 0,01 mg à 1 mg par kg de poids corporel est toutefois préféré.

Les compositions pharmaceutiques selon l'invention peuvent être utilisées en médecine interne, par exemple dans le traitement d'états pathologiques organiques, tels que l'hypertension artérielle et les coronarites, accompagnés et aggravés par un état anxieux; en médicine psychosomatique, par exemple pour le traitement de l'asthme, des ulcères gastro-duodénaux, des colopaties et d'autres affections digestives fonctionnelles, ainsi qu'en psychiatrie, par exemple pour les traitements d'états d'agitation anxieux chez les sujets psychotiques.

Bien entendu, la présente invention ne se trouve pas limitée aux exemples particuliers mentionnés à simple titre illustratif, mais il est parfaitement possible, sans pour autant sortir du cadre de l'invention, d'en imaginer un certain nombre de variantes et de modifications. Il est en particulier possible d'associer aux composés de formule générale I de la présente invention d'autres principes actifs venant compléter ou renforcer leur action thérapeutique.

**Revendications**

1. Nouveaux dérivés d'orthochloro benzoyl-2 chloro-4 glycylanilides répondant à la formule générale I:

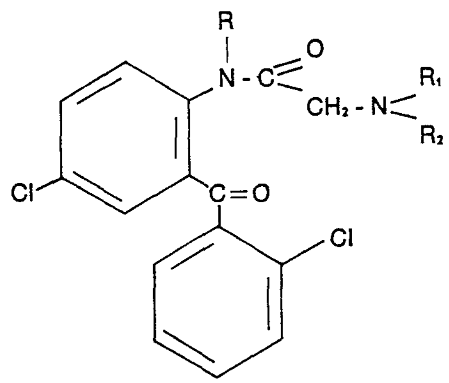

(I)

dans laquelle:

R représente un groupe alcoyle inférieur et plus particulièrement le radical méthyle;

$R_1$ représente un hydrogène, un alcoyle, ou un hydroxyalcoyle;

$R_2$ représente un hydroxyalcoyle, un alcényle, un alcynyle éventuellement substitués une ou plusieurs fois par un radical alcoyle, ou un cycloalcoyle de 3 à 6 atomes de carbone éventuellement substitué par un radical alcoyle;

$R_1$ et $R_2$ peuvent également former avec l'atome d'azote auquel ils sont fixés un hétérocycle azoté contenant éventuellement un second hétéroatome choisi parmi l'oxygène et l'azote;

ainsi que leurs sels obtenus avec des acides minéraux ou organiques thérapeutiquement acceptables.

2. Le N-N' diméthyl N(hydroxy-2 éthyl) ortho chloro benzoyl-2' chloro-4' glycylanilide, répondant à la formule générale I selon la revendication 1.

3. Le N-N bis (hydroxy-2 éthyl) N' méthyl ortho chloro benzoyl-2' chloro-4' glycylanilide, répondant à la formule générale I selon la revendication 1.

4. Le N(diméthyl-1-1 propargyl) N' méthyl ortho chloro benzoyl-2' chloro-4' glycylanilide, répondant à la formule générale I selon la revendication 1.

5. Le N-cyclopropyl N' méthyl ortho chloro benzoyl-2' chloro-4' glycylanilide, répondant à la formule générale I selon la revendication 1.

6. Le N-cyclopentyl N' méthyl ortho chloro benzoyl-2' chloro-4' glycylanilide, répondant à la formule générale I selon la revendication 1.

7. Le N-cyclohexyl N' méthyl ortho chloro benzoyl-2' chloro-4' glycylanilide, répondant à la formule générale I selon la revendication 1.

8. Le N-cyclohexyl N-N' diméthyl ortho chloro benzoyl-2' chloro-4' glycylanilide, répondant à la formule générale I selon la revendication 1.

9. Le N-méthyl ortho chloro benzoyl-2' chloro-4' morpholino-2 acétanilide, répondant à la formule générale I selon la revendication 1.

10. Le N-méthyl N' (méthyl-2 allyl) ortho chloro benzoyl-2' chloro-4' glycylanilide, répondant à la formule générale I selon la revendication 1.

11. Nouveaux dérivés de formule générale I selon la revendication 1, caractérisés en ce qu'ils

se présentent sous la forme de sels des composés selon l'une des revendications 2 à 10, avec des acides minéraux ou organiques thérapeutiquement acceptables, tels que les chlorhydrates et les maléates acides.

12. Procédé de préparation des composés de formule générale I selon l'une des revendications 1 à 11, caractérisé par le fait qu'il consiste à faire réagir un halo-acétamide de formule générale II

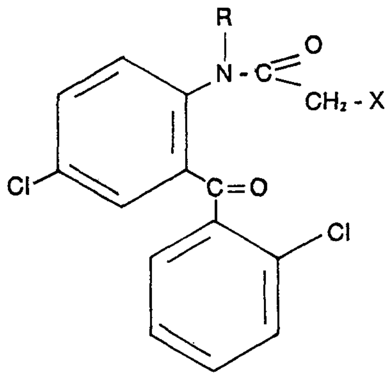

(II)

dans laquelle:

R a la signification donnée à la revendication 1 et

X représente un atome d'halogène

avec un amine de formule III

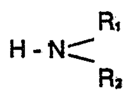

(III)

dans laquelle:

$R_1$ et $R_2$ ont les significations données à la revendication 1.

13. Procédé selon la revendication 12, caractérisé en ce que l'halo-acétamide de formule II est obtenu par réaction d'une n-alcoylamino benzophénone de formule générale

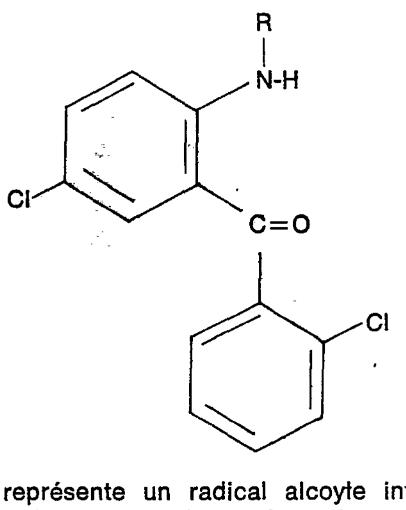

où R représente un radical alcoyle inférieur, avec un halogénure d'halogénoacétyle de formule

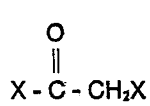

où X représente un atome d'halogène.

14. A titre de médicaments nouveaux, les composés de formule générale I selon l'une des revendications 1 à 11, ainsi que leurs sels d'addition avec des acides physiologiquement acceptables.

15. Les compositions pharmaceutiques contenant comme principes actifs au moins un composé de formule générale I selon l'une des revendications 1 à 11, ou l'un de leurs sels d'addition avec des acides physiologiquement acceptables.

16. Les compositions pharmaceutiques selon la revendication 15, dans lesquelles aux composés de formule générale I, peuvent être associés d'autres principes actifs venant compléter ou renforcer leur action thérapeutique.

**Patentansprüche**

1. Neue 2-Orthochlorbenzoyl-4-chlor-glycinanilide der allgemeinen Formel I:

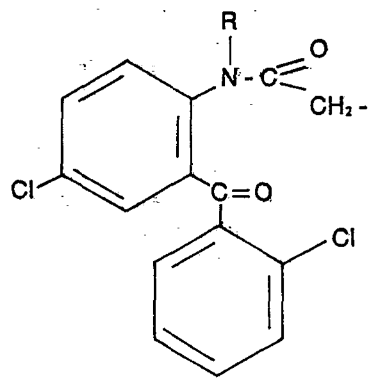

(I)

in welcher

R eine nied. Alkylgruppe, insbesondere die Methylgruppe ist;

$R_1$ Wasserstoff, Alkyl oder Hydroxyalkyl darstellt;

$R_2$ Hydroxyalkyl, Alkenyl, Alkinyl, gegebenenfalls ein- oder mehrfach substituiert durch einen Alkylrest, oder Cycloalkyl mit 3 bis 6 Kohlenstoffatomen, gegebenenfalls durch einen Alkylrest substituiert, ist;

$R_1$ und $R_2$ aber auch zusammen mit dem Stickstoffatom, an das sie gebunden sind, einen stickstoffhaltigen Heterocyclus bilden können, der gegebenenfalls ein zweites Heteroatom, ausgewählt unter Sauerstoff und Stickstoff, enthält;

sowie deren Salze mit Mineralsäuren oder organischen therapeutisch annehmbaren Säuren.

2. N,N'-Dimethyl-N-(2-hydroxyäthyl)-2'-orthochlorbenzoyl-4'-chlor-glycinanilid entsprechend der allgemeinen Formel I gemäss Anspruch 1.

3. N,N-Bis-(2-hydroxyäthyl)-N'-methyl-2'-orthochlorbenzoyl-4'-chlor-glycinanilid entsprechend der allgemeinen Formel I gemäss Anspruch 1.

4. N-(1,1-Dimethylpropargyl)-N'-methyl-2'-orthochlorbenzoyl-4'-chlorglycinanilid entsprechend der allgemeinen Formel I gemäss Anspruch 1.

5. N-Cyclopropyl-N'-methyl-2'-orthochlorben-

zoyl-4'-chlorglycinanilid entsprechend der allgemeinen Formel I gemäss Anspruch 1.

6. Cyclopentyl-N'-methyl-2'-orthochlorbenzoyl--4'-chlorglycinanilid entsprechend der allgemeinen Formel I gemäss Anspruch 1.

7. N-Cyclohexyl-N'-methyl-2'-orthochlorbenzoyl-4'-chlorglycinanilid entsprechend der allgemeinen Formel I gemäss Anspruch 1.

8. N-Cyclohexyl-N,N'-dimethyl-2'-orthochlorbenzoyl-4'-chlorglycinanilid entsprechend der allgemeinen Formel I gemäss Anspruch 1.

9. N-Methyl-2'-orthochlorbenzoyl-4'-chlor-2--morpholino-acetanilid entsprechend der allgemeinen Formel I gemäss Anspruch 1.

10. N-Methyl-N'-(2-methylallyl)-2'-orthochlorbenzoyl-4'-chlorglycinanilid entsprechend der allgemeinen Formel I gemäss Anspruch 1.

11. Neue Derivate der allgemeinen Formel I gemäss Anspruch 1, dadurch gekennzeichnet, dass sie in Form von Salzen der Verbindungen gemäss einem der Ansprüche 2 bis 10 mit Mineralsäuren oder organischen therapeutisch annehmbaren Säuren vorliegen, wie die Chlorhydrate und die sauren Maleate.

12. Verfahren zur Herstellung der Verbindungen der allgemeinen Formel I gemäss einem der Ansprüche 1 bis 11, dadurch gekennzeichnet, dass man ein Halogenacetamid der allgemeinen Formel II

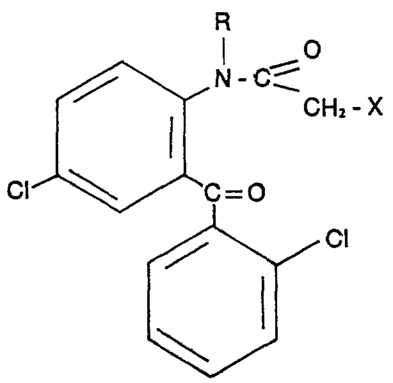

(II)

in welcher
R die in Anspruch 1 angegebene Bedeutung hat und
X ein Halogenatom darstellt,
mit einem Amin der Formel III

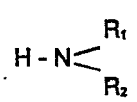

(III)

in welcher
$R_1$ und $R_2$ die in Anspruch 1 angegebenen Bedeutungen haben, reagieren lässt.

13. Verfahren gemäss Anspruch 12, dadurch gekennzeichnet, dass das Halogenacetamid der Formel II durch Umsetzung eines N-Alkylaminobenzophenons der allgemeinen Formel

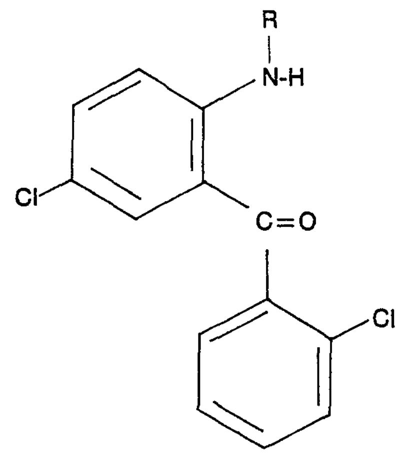

worin R einen nied. Alkylrest bedeutet, mit einem Halogenacetylhalogenid der Formel

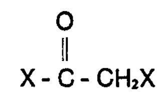

worin X ein Halogenatom darstellt, erhalten wurde.

14. Die Verbindungen der allgemeinen Formel I gemäss einem der Ansprüche 1 bis 11 sowie ihre Additionssalze mit physiologisch annehmbaren Säuren als neue Medikamente.

15. Pharmazeutische Zusammensetzungen, die als Wirkstoffe mindestens eine Verbindung der allgemeinen Formel I gemäss einem der Ansprüche 1 bis 11 oder eines ihrer Additionssalze mit physiologisch annehmbaren Säuren enthalten.

16. Pharmazeutische Zusammensetzungen gemäss Anspruch 15, in welchen die Verbindungen der allgemeinen Formel I mit anderen Wirkstoffen assoziiert sein können, was zu einer Komplettierung oder Verstärkung der therapeutischen Wirkung derselben führt.

**Claims**

1. New-o-chloro-2-benzoyl-4-chloroglycyl anilide derivatives corresponding to the following general formula:

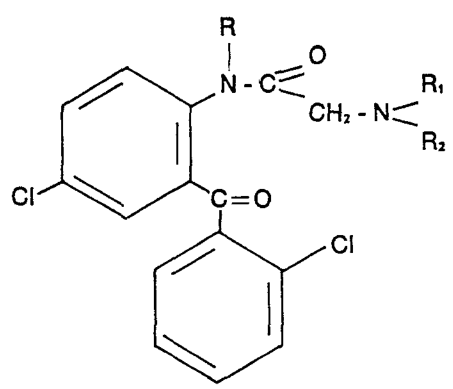

(I)

wherein
R represents a lower alkyl group, preferably methyl;
$R_1$ represents hydrogen, alkyl, or hydroxyalkyl;

$R_2$ represents hydroxyalkyl, alkenyl or alkynyl groups which may be substituted one or more times by an alkyl radical, or a from 3- to 6-membered cycloalkyl group optionally substituted by an alkyl radical;

$R_1$ and $R_2$, together with the nitrogen atom to which they are attached, may complete a nitrogen heterocycle optionally containing a second hetero atom selected from oxygen and nitrogen;

and salts thereof with therapeutically acceptable mineral or organic acids.

2. The N,N'-dimethyl-N-(2-hydroxyethyl)-o-chloro-2'-benzoyl-4'-chloroglycyl anilide according to formula I of claim 1.

3. The N,N-bis-(2-hydroxyethyl)-N'-methyl-o-chloro-2'-benzoyl-4'-chloroglycyl anilide according to formula I of claim 1.

4. The N-(1,1-dimethylpropargyl)-N'-methyl-o-chloro-2'-benzoyl-4'-chloroglycyl anilide according to formula I of claim 1.

5. The N-cyclopropyl-N'-methyl-o-chloro-2'-benzoyl-4'-chloroglycyl anilide according to formula I of claim 1.

6. The N-cyclopentyl-N'-methyl-o-chloro-2'-benzoyl-4'-chloroglycyl anilide according to formula I of claim 1.

7. The N-cyclohexyl-N'-methyl-o-chloro-2'-benzoyl-4'-chloroglycyl anilide according to formula I of claim 1.

8. The N-cyclohexyl-N,N'-dimethyl-o-chloro-2'-benzoyl-4'-chloroglycyl anilide according to formula I of claim 1.

9. The N-methyl-o-chloro-2'-benzoyl-4'-chloro-2-morpholino-acetanilide according to formula I of claim 1.

10. The N-methyl-N'-(2-methylallyl)-o-chloro-2'-benzoyl-4'-chloroglycyl anilide according to formula I of claim 1.

11. New compounds corresponding to general formula I of claim 1, characterised in that they are in the form of salts of compounds according to any of claims 2 to 10, with therapeutically acceptable mineral or organic acids, such as hydrochlorides and the acid maleates.

12. A process for the preparation of the compounds corresponding to general formula I claimed in any of claims 1 to 11, characterised in that a haloacetamide corresponding to the following general formula:

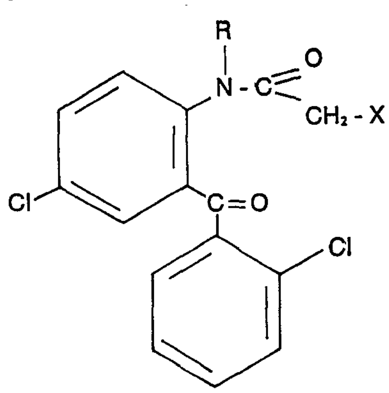

(II)

wherein

R is as defined in claim 1 and

X represents a halogen atom,

is reacted with an amine corresponding to the following general formula:

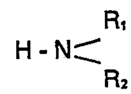

(III)

wherein

$R_1$ and $R_2$ are as defined above in claim 1.

13. A process according to claim 12, characterised in that the halo-acetamide of the general formula II is obtained by reaction of N-alkylamino-benzophenone corresponding to the general formula:

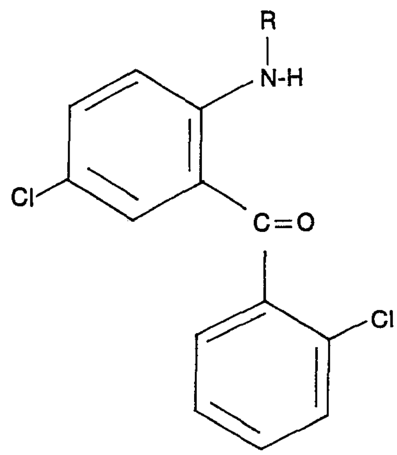

wherein

R is a lower alkyl radical group,

with an halide haloacetyl corresponding to the general formula:

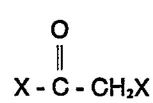

wherein

X represents an halogen atom.

14. As new medicaments, the compounds corresponding to formula I claimed in any of claims 1 to 11 and the addition salts with physiologically acceptable acids.

15. The pharmaceutical compositions containing as active principles at least one compound corresponding to general formula I as claimed in any of claims 1 to 11 or one of the addition salts thereof with physiologically acceptable acids.

16. The pharmaceutical compositions claimed in claim 15 in which other active principles may be associated with the compounds corresponding to general formula I to complete or strengthen the therapeutic action thereof.